# FASCICULE DE BREVET EUROPEEN

(11) **EP 1 237 531 B1**
(45) Date de publication et mention de la délivrance du brevet: **28.02.2007**
(21) Numéro de dépôt: 00981415.3
(22) Date de dépôt: 10.11.2000
(51) Int. Cl.: A61K 8/22, A61K 8/86, A61Q 5/08

(54) **COMPOSITION DE DECOLORATION POUR FIBRES KERATINIQUES COMPRENANT UN POLYMERE EPAISSISSANT A SQUELETTE AMINOPLASTE-ETHER**
ZUSAMMENSETZUNG ZUM ENTFÄRBEN VON KERATINFASERN, DIE EIN VERDICKENDES POLYMER MIT AMINOPLAST- GRUNDGERÜST ENTHÄLT
BLEACHING COMPOSITION FOR KERATINIC FIBRES, CONTAINING A THICKENING POLYMER WITH AN ETHER PLASTIC SKELETON

(30) Priorité: 08.12.1999 FR 9915484
(43) Date de publication de la demande: 11.09.2002
(73) Titulaire: L'ORÉAL, 75008 Paris (FR)
(72) Inventeur: LEGRAND, Frédéric, F-92400 Courbevoie (FR); ALLARD, Delphine, F-92700 Colombes (FR)
(74) Mandataire: Miszputen, Laurent
(86) Numéro de dépôt international: PCT/FR2000/003141
(87) Numéro de publication internationale: WO 2001/041725

(56) Documents cités:
- EP-A- 0 958 807
- FR-A- 2 770 522

## Description

La présente invention concerne une composition pour la décoloration des fibres kératiniques et en particulier des fibres kératiniques humaines telles que les cheveux, comprenant au moins un agent oxydant et au moins un polymère épaississant à squelette aminoplaste-éther.

Il est connu de décolorer les fibres kératiniques et en particulier les cheveux humains, avec des compositions de décoloration contenant un ou plusieurs agents oxydants. Parmi les agents oxydants classiquement utilisés, on peut citer le peroxyde d'hydrogène ou des composés susceptibles de produire le peroxyde d'hydrogène par hydrolyse, tels que le peroxyde d'urée ou les persels comme les perborates, les percarbonates et les persulfates, le peroxyde d'hydrogène et les persulfates étant particulièrement préférés.

Lesdites compositions de décoloration se présentent principalement sous forme de produits anhydres ( poudres ou crèmes ) contenant des composés alcalins (amines et silicates alcalins), et un réactif peroxygéné tels que les persulfates, perborates ou percarbonates, d'ammonium ou de métaux alcalins, que l'on dilue au moment de l'emploi avec une composition aqueuse de peroxyde d'hydrogène.
Les compositions de décoloration peuvent aussi résulter du mélange, au moment de l'emploi, de la poudre anhydre de réactif péroxygéné avec une composition aqueuse contenant les composés alcalins et une autre composition aqueuse contenant le peroxyde d'hydrogène.
Les compositions de décoloration se présentent également sous forme de compositions aqueuses épaissies de peroxyde d'hydrogène, prêtes à l'emploi.

Par "composition prête à l'emploi" on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

Pour localiser le produit de décoloration à l'application sur les cheveux afin qu'il ne coule pas sur le visage ou en dehors des zones que l'on se propose de décolorer, on a jusqu'ici eu recours à l'emploi d'épaississants traditionnels tels que l'acide polyacrylique réticulé, les hydroxyéthylcelluloses, certains polyuréthanes, les cires, et en outre, dans le cas des compositions aqueuses de décoloration, à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les systèmes épaississants mentionnés ci-dessus ne permettaient pas d'obtenir des décolorations suffisamment puissantes et homogènes et laissaient les cheveux rèches.
Par ailleurs, elle a également constaté que les compositions de décoloration prêtes à l'emploi contenant le ou les agents oxydants, et en outre les systèmes épaississants de l'art antérieur ne permettaient pas une application suffisamment précise sans coulures ni chutes de viscosité dans le temps.

Or, après d'importantes recherches menées sur la question, la Demanderesse vient maintenant de découvrir qu'il est possible d'obtenir des compositions de décoloration prêtes à l'emploi qui ne coulent pas et restent donc bien localisées au point d'application, et qui permettent aussi d'obtenir des décolorations puissantes et homogènes tout en laissant les cheveux moins rèches si on introduit dans la composition une quantité efficace d'un polymère à squelette aminoplaste-éther.

Ces découvertes sont à la base de la présente invention.

La présente invention a ainsi pour objet une composition prête à l'emploi pour la décoloration des fibres kératiniques, en particulier des fibres kératiniques humaines, telles que les cheveux, comprenant, dans un milieu approprié pour la décoloration, au moins un agent oxydant, qui est caractérisée par le fait qu'elle contient en outre au moins un polymère à squelette aminoplaste-éther.

Selon l'invention, ladite composition est anhydre ou aqueuse.
Lorsque la composition prête à l'emploi selon l'invention résulte du mélange extemporané de plusieurs compositions, le polymère à squelette aminoplaste-éther peut être présent dans une ou plusieurs ou dans la totalité des compositions mélangées.
De la sorte, le polymère à squelette aminoplaste-éther peut être présent dans une composition anhydre sous forme de poudre de préférence pulvérulente ou de crème et/ou dans une ou plusieurs compositions aqueuses.

De préférence selon l'invention, le polymère à squelette aminoplaste-éther est présent dans au moins une composition aqueuse que l'on mélange au moment de l'emploi avec une composition anhydre sous forme de poudre ou de crème contenant au moins un agent oxydant.
Plus préférentiellement encore, l'une de ces compositions aqueuses mélangée à la composition anhydre renferme du peroxyde d'hydrogène.

L'invention vise également un procédé de décoloration des fibres kératiniques, et en particulier des fibres kératiniques humaines telles que les cheveux utilisant la composition de décoloration prête à l'emploi telle que décrite selon l'invention.

L'invention vise aussi des dispositifs de décoloration ou "Kits" d'emballage contenant une telle composition prête à l'emploi.

Ainsi, un dispositif à deux compartiments comprend un premier compartiment contenant au moins une poudre ou une crème anhydre ou une composition aqueuse, et le deuxième compartiment une composition aqueuse, l'un au moins des deux compartiments contenant au moins un agent oxydant et l'un au moins des deux compartiments contenant au moins un polymère à squelette aminoplaste-éther.

Un autre « kit » à plusieurs compartiments peut être constitué d'un premier compartiment contenant une poudre ou une crème anhydre et de deux autres compartiments contenant chacun une composition aqueuse, l'un au moins des trois compartiments contenant au moins un agent oxydant et l'un au moins des trois compartiments contenant au moins un polymère à squelette aminoplaste-éther.

Mais d'autres caractéristiques, aspects, objets et avantages de l'invention apparaîtront encore plus clairement à la lecture de la description et des exemples qui suivent.

Par aminoplaste-éther, on entend au sens de la présente invention, tout produit issu de la condensation d'un aldéhyde avec une amine ou un amide.
Par aminoplaste-éther, on entend aussi au sens de la présente invention, toute unité structurale formée d'un résidu aminoplaste et d'un résidu hydrocarboné divalent lié au résidu aminoplaste par une liaison éther.

Les polymères à squelette aminoplaste-éther utilisés selon l'invention, sont choisis parmi ceux contenant au moins un motif de structure (I) suivante : 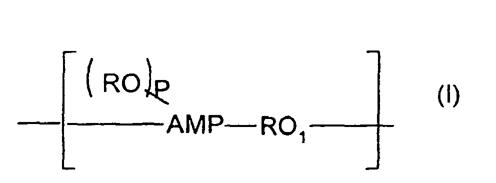 dans laquelle :
- AMP est un résidu aminoplaste avec unités alkylènes,
- R désigne un atome d'hydrogène, un radical alkyl C₁C₄ ou un radical acyle C₁C₄,
- RO₁ est un résidu alkylèneoxy divalent,
- p désigne un nombre entier positif,
le ou les groupements OR étant liés aux unités alkylènes du résidu AMP ; et ceux contenant au moins un motif de structure (II) suivante : 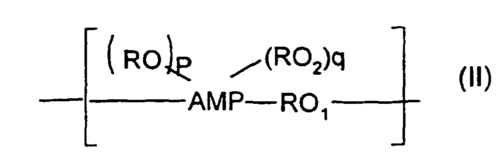 dans laquelle :
- AMP, R, RO₁ et p ont la même signification que précédemment,
- RO₂ est un groupe hydrophobe différent de RO lié à AMP via un hétéroatome et comprenant au moins deux atomes de carbone, et,
- q est un nombre entier positif ;
les polymèresétantde formules (III) et (III)bis suivantes : 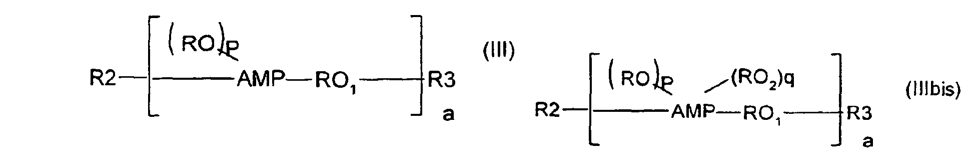 dans lesquelles :
AMP, R, RO₁, RO₂, p et q ont la même signification que précédemment, R2 ou R3, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
a étant un nombre plus grand que 1 et de préférence plus grand que 2 ;

Les résidus aminoplastes porteurs de leurs groupements OR sont choisis parmi ceux de structure (XVI) suivante : 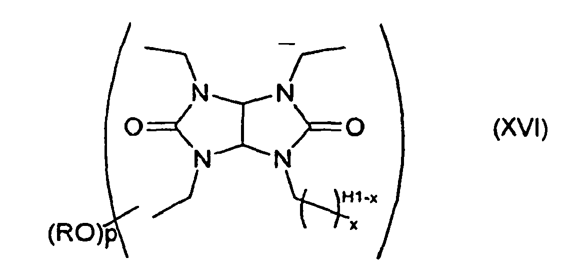 dans laquelle R, p, x désign ont les mêmes significations que précédemment ; 0 ou 1 ;
Les résidus alkylèneoxy divalents sont ceux correspondants aux diols de formule générale (XVII) suivante :

   HO-(ZO)y-(Z'O)y'-Z3OH (XVII),
y et y' étant des nombres allant de 0 à 1000,
et Z, Z' et Z3 désignent -CH2CH2-, et l'un au moins de y ou y' est différent de 0. Les composés de formule (XVII) sont alors des polyéthylèneglycols.

Les polymères de formule (I) selon l'invention sont en particulier décrits dans le brevet US 5 914 373 dont le contenu fait partie intégrante de l'invention.

Comme polymères à squelette aminoplaste-éther de formule (I), on peut en particulier citer les produits PURE-THIX L [PEG-180/Octoxynol-40/TMMG Copolymer (Nom INCI)], PURE-THIX M [PEG-180/Laureth-50/TMMG Copolymer (Nom INCI)], et PURE-THIX HH [Polyether-1 (Nom INC1)] vendus par la société SUD-CHEMIE.

Les polymères à squelette aminoplaste-éther sont utilisés de préférence en une quantité pouvant varier d'environ 0,01 à 10% en poids du poids total de la composition prête à l'emploi. Plus préférentiellement, cette quantité varie d'environ 0,1 à 5% en poids.

Les agents oxydants utilisables selon l'invention sont choisis de préférence parmi le peroxyde d'hydrogène et les composés libérant du peroxyde d'hydrogène par hydrolyse, tels que le peroxyde d'urée et les persels.
Comme persels on peut utiliser selon l'invention les persulfates et les perborates et en particulier le persulfate de sodium ou le persulfate de potassium.
Comme autres agents oxydants on peut citer les chlorites.
On peut aussi utiliser un système enzymatique générateur d'espèces oxydantes et en particulier de peroxyde d'hydrogène. Comme exemple de tels systèmes enzymatiques, on peut citer les oxydoréductases à deux électrons associées à leur donneur en présence d'air, et plus particulièrement le système uricase, acide urique et air.

On peut aussi utiliser des peroxydes organiques.
La concentration en peroxyde d'hydrogène des compositions prêtes à l'emploi peut varier de 2 à 40 volumes. Celle des autres composés oxydants dont en particulier les composés susceptibles de former du peroxyde d'hydrogène par hydrolyse peut varier de 0,1 à 25 % en poids environ par rapport au poids total de la composition.
La composition selon l'invention peut encore contenir, en plus des agents oxydants définis ci-dessus, des colorants directs. Ces colorants directs peuvent notamment alors être choisis parmi les colorants nitrés, azoïques ou anthraquinoniques, qu'ils soient neutres, acides ou cationiques.

Plus particulièrement, les compositions selon l'invention peuvent en outre contenir, au moins un polymère substantif cationique ou amphotère.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (I), (II), (III) ou (IV) suivantes: 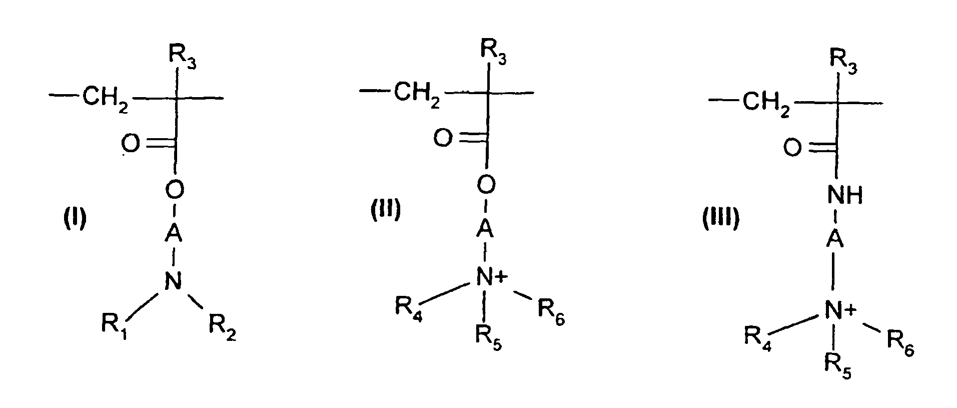 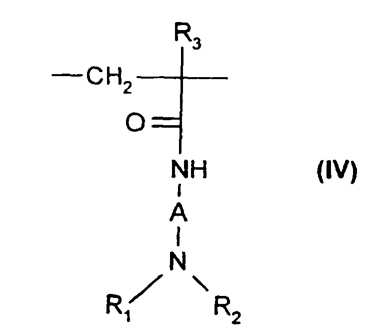 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100 par la société CIBA GEIGY,
   - le copolymère d'acrylamide et de méthosulfate de méthacryloyloxyéthyltriméthylammonium vendu sous la dénomination RETEN par la société HERCULES,
   - les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713 par la société ISP,
   - les copolymère vinylpyrrolidone / méthacrylamidopropyl dimethylamine commercialisés notamment sous la dénomination STYLEZE CC 10 par ISP,
   - et les copolymères vinylpyrrolidone / méthacrylamide de diméthylaminopropyle quaternisés tel que le produit vendu sous la dénomination "GAFQUAT HS 100" par la société ISP.
(2) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(6) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(7) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 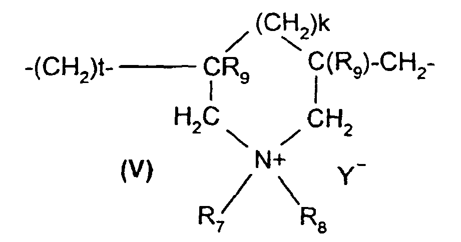 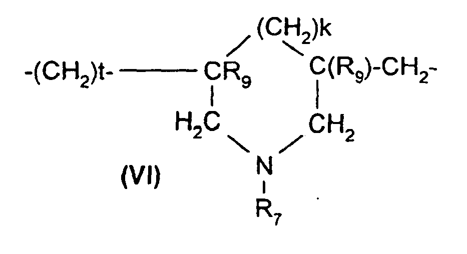 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(10) Le polymère de diammonium quaternaire contenant des motifs récurrents répondant à la formule : 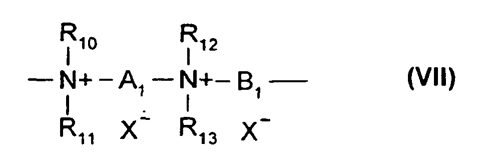 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁,
   R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ; A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
      De préférence, X- est un anion tel que le chlorure ou le bromure.
      Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
      On peut utiliser plus particulièrement les polymères qui sont constitués de motifs récurrents répondant à la formule (VIII) suivante: 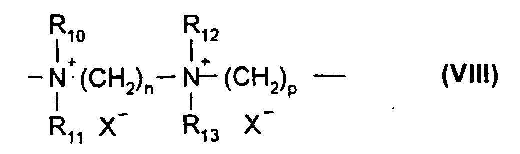 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
(11) Les polymères de polyammonium quaternaire constitués de motifs de formule (IX) : 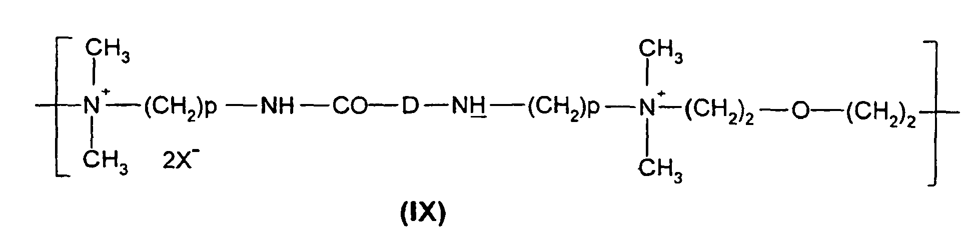 dans laquelle :
   p désigne un nombre entier variant de 1 à 6 environ,
   D peut être nul ou peut représenter un groupement -(CH₂)ᵣ-CO- dans
   lequel r désigne un nombre égal à 4 ou à 7, et
   X- est un anion dérivé d'un acide minéral ou organique.
      Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689, 4 702 906, 4719282.
      Parmi ces polymères, on préfère ceux de masse moléculaire mesurée par RMN du Carbone 13 inférieure à 100000, et dans la formule de laquelle :
      p est égal à 3, et,
         a) D représente un groupement -(CH₂)₄-CO-, X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1,
         b) D représente un groupement -(CH₂)₇-CO-, X désigne un atome de chlore, la masse moléculaire mesurée par RMN du Carbone 13 ( RMN¹³C ) étant d'environ 8100 ; un polymère de ce type est proposé par la société MIRANOL sous le nom de MIRAPOL-AZ1,
         c) D désigne la valeur zéro, X désigne un atome de chlore, la masse moléculaire mesurée par RMN du Carbone 13, ( RMN¹³C ) étant d'environ 25500 un polymère de ce type est vendu par la société MIRANOL sous le nom MIRAPOL-A15,
         d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).
         Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13, (RMN¹³C) étant d'environ 25500.
(12) Les polymères quaternaires de vinylpyrrolidone et de vinylimidazole tels que par exemple les produits commercialisés sous les dénominations Luviquat FC 905, FC 550 et FC 370 par la société B.A.S.F.
(13) Les polyamines comme le Polyquart H vendu par HENKEL, référencé sous le nom de " POLYETHYLENEGLYCOL (15) TALLOW POLYAMINE " dans le dictionnaire CTFA.
(14) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères de formules (W) et (U) suivantes : 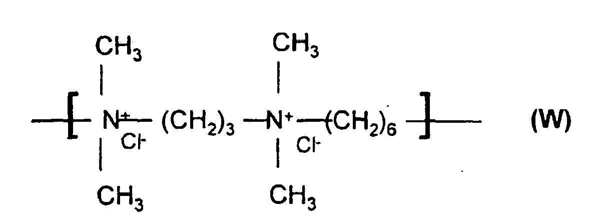 et notamment ceux dont le poids moléculaire, déterminé par chromatographie par perméation de gel, est compris entre 9500 et 9900; 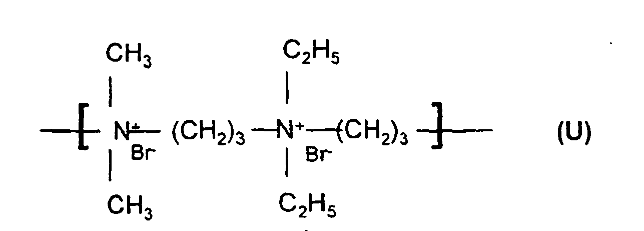 et notamment ceux dont le poids moléculaire, déterminé par chromatographie par perméation de gel, est d'environ 1200.

La concentration en polymère cationique dans les compositions selon la présente invention peut varier de 0,01 à 10% en poids par rapport au poids total de la composition, de préférence de 0,05 à 5% et plus préférentiellement encore de 0,1 à 3%.

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes; K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères filmogènes amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle. Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique. Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 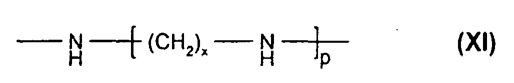 où x=2 et p=2 ou 3, ou bien x=3 et p=2 ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 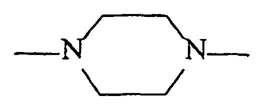
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 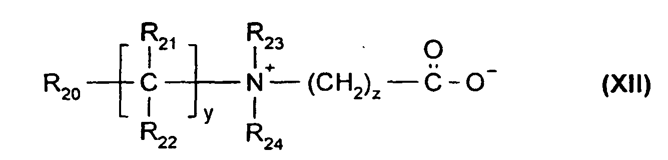 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl-carboxyméthylammonio-éthylméthacrylate de méthyle tel que le produit vendu sous la dénomination DIAFORMER Z301 par la société SANDOZ.
(5) les polymères dérivés du chitosane comportant des motifs monomères répondant aux formules (XIII), (XIV), (XV) suivantes : 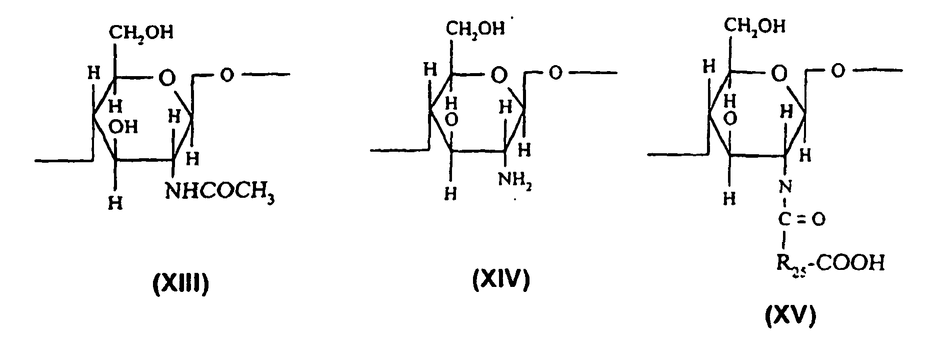 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 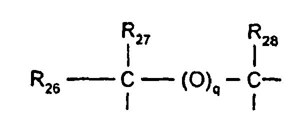 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 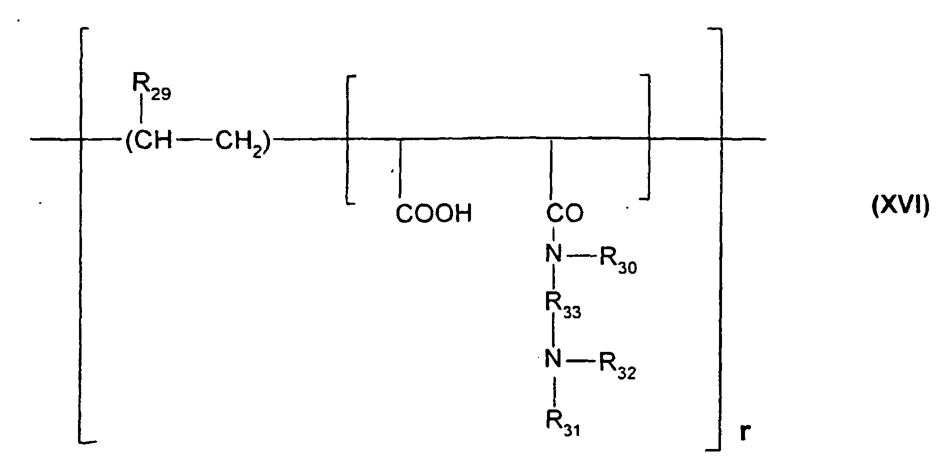 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂-, -CH₂-CH₂-CH₂-, -CH₂-CH(CH₃)-, R₃₁ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 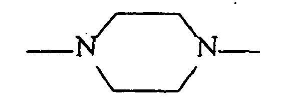 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 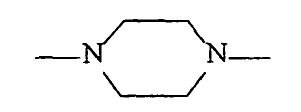 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs.
Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s):

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, a.-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alky((C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser les acides d'alkyl D galactoside uroniques et leurs sels, les acides alkyl(C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s):

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras : les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₃₄ -CONHCH₂CH₂ -N(R₃₅)(R₃₆)(CH₂COO⁻)

dans laquelle : R₃₄ désigne un radical alkyle d'un acide R₃₄-COOH présent dans l'huile de coprah hydrolysée, un radical heptyle, nonyle ou undécyle, R₃₅ désigne un groupement bêta-hydroxyéthyle et R₃₆ un groupement carboxyméthyle ; et

R₃₄'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₃₄' désigne un radical alkyle d'un acide R₃₇ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition prête à l'emploi selon l'invention peuvent varier de 0,01 à 40% et de préférence de 0,1 à 30% du poids total de la composition.

Les compositions selon l'invention peuvent également contenir d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique et les polymères associatifs ioniques ou non ioniques tels que les polymères commercialisés sous les appellations PEMULEN TR1 ou TR2 par la société GOODRICH, SALCARE SC90 par la société ALLIED COLLOIDS, ACULYN 22, 28, 33, 44, ou 46 par la société ROHM & HAAS et ELFACOS T210 et T212 par la société AKZO.

Ces épaississants d'appoint peuvent représenter de 0,05 à 10% en poids du poids total de la composition.

Les compositions selon l'invention contiennent en outre avantageusement un agent alcalin.
Parmi les agents alcalinisants on peut citer, à titre d'exemple, l'ammoniaque, le chlorure d'ammonium, les carbonates alcalins ou alcalino-terreux, les silicates alcalins ou alcalino-terreux, les phosphates alcalins ou alcalino-terreux, les alcanolamines telles que les mono-, di- et triéthanolamines ainsi que leurs dérivés, les hydroxydes de sodium ou de potassium et les composés de formule (XIX) suivante : 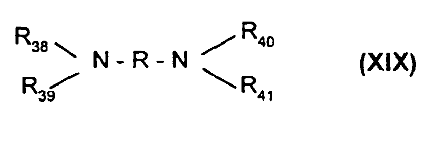 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les compositions de l'invention peuvent également contenir des agents séquestrants comme par exemple l'acide éthylènediamine tétraacétique (EDTA).

Lorsque les compositions contenant l'agent oxydant et le polymère à squelette aminoplaste-éther sont sous forme anhydre ( poudre ou crème ), elles contiennent les agents principaux et additifs mentionnés ci-dessus sous forme de solides ou de liquides essentiellement anhydres. Elles peuvent également contenir des charges minérales ou organiques telles que la silice ou les argiles. Elles peuvent aussi contenir des liants tels que la vinylpyrrolidone, les huiles ou les cires, les polyalkylèneglycols ou les dérivés de polyalkylèneglycols. Elles peuvent aussi contenir des lubrifiants comme les stéarates de polyol ou les stérates de métaux alcalins ou alcalino-terreux, ainsi que des agents colorants ou matifiants comme les oxydes de titane.

Lorsque le milieu contenant l'agent oxydant est un milieu aqueux, il peut éventuellement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition de décoloration selon l'invention encore contenir une quantité efficace d'autres agents, par ailleurs antérieurement connus en décoloration, tels que divers adjuvants usuels comme des silicones volatiles ou non, cycliques ou linéaires ou ramifiées, organomodifiées (notamment par des groupements amines) ou non organomodifiées, des conservateurs, des céramides, des cires ou des huiles, végétales, minérales ou synthétiques, des acides et en particuliers des AHA etc...

Bien entendu, l'homme de l'art veillera à choisir le ou les éventuels composés complémentaires mentionnés ci-avant, de manière telle que les propriétés avantageuses attachées intrinsèquement à la composition de décoloration selon l'invention ne soient pas, ou substantiellement pas, altérées par la ou les adjonctions envisagées.

Le pH de la composition prête à l'emploi est généralement compris entre les valeurs 4 et 12. Il est de préférence compris entre 7 et 11,5 et encore plus préférentiellement entre 8 et 11.

De préférence, le procédé de décoloration selon l'invention consiste à appliquer la composition oxydante prête à l'emploi, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Des exemples concrets illustrant l'invention sont indiqués ci-après, sans pour autant présenter un caractère limitatif.

### EXEMPLE 1 :

On a préparé la composition de décoloration aqueuse prête à l'emploi suivante (exprimée en grammes):

| | |
|---|---|
| Peroxyde d'hydrogène à 200 volumes | 12 |
| Stabilisant | qs |
| PURE-THIX-HH (INCI=POLYETHER-1) | 0,3 MA* |
| Agent de pH qs | pH 4,7 |
| Eau qsp | 100 |

| | |
|---|---|
| MA*= Matière Active | |

La composition de décoloration ci-dessus a été appliquée et laissée 45 minutes sous casque sur des cheveux naturels, puis rincée abondamment à l'eau. On a obtenu un éclaircissement régulier de la chevelure.

### EXEMPLE 2 :

On a préparé la composition de décoloration suivante (exprimée en grammes):

| **Composition anhydre** | |
|---|---|
| Persulfate de potassium | 35 |
| Persulfate de sodium | 30 |
| Métasilicate de sodium | 14 |
| Chlorure d'ammonium | 5 |
| EDTA | 1 |
| Dioctylsulfosuccinate de sodium/benzoate de sodium | 1 |
| Stéarate de calcium | 1 |
| PURE-THIX-HH (INCI=POLYETHER-1) | 3 |
| Silice | 7 |

40 g de la composition anhydre ci-dessus ont été mélangés avec 80 g de la composition aqueuse suivante :

| **Composition aqueuse** | |
|---|---|
| Alcool cétéarylique/ceteareth 30 | 2,85 |
| Stabilisants | 0,06 |
| Séquestrant | 0,15 |
| Peroxyde d'hydrogène à 200 volumes | 9 |
| Acide phosphorique qsp | pH2 |
| Eau distillée qsp... | 100 |

On a obtenu alors une crème décolorante prête à l'emploi qui, appliquée et laissée 45 minutes sous casque, a permis d'obtenir une décoloration puissante et homogène de cheveux naturels foncés.

## Revendications

1. Composition prête à l'emploi pour la décoloration des fibres kératiniques, en particulier des fibres kératiniques humaines telles que les cheveux, comprenant, dans un milieu approprié pour la décoloration, au moins un agent oxydant, **caractérisée par le fait qu'**elle contient en outre au moins un polymère épaississant à squelette aminoplaste-éther choisi parmi ceux contenant au moins un motif de structure (1) suivante : 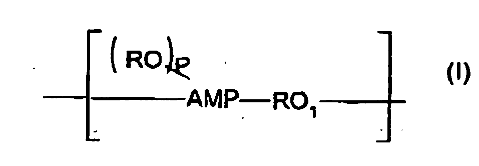 dans laquelle :
- AMP est un résidu aminoplaste avec unités alkylènes.
- R désigne un atome d'hydrogène, un radical alkyl C₁C₄ ou un radical acyle C₁C₄,
- RO₁ est un résidu alkylèneoxy divalent,
- p désigne un nombre entier positif,
le ou les groupements OR étant liés aux unités alkylènes du résidu AMP ; et ceux contenant au moins un motif de structure (II) suivante : 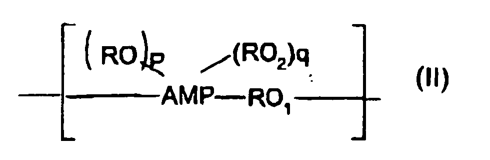 dans laquelle:
- AMP. R, RO₁ et p ont la même signification que ci-dessus,
- RO₂ est un groupe hydrophobe différent de RO lié à AMP via un hétéroatome et comprenant au moins deux atomes de carbone, et.
- q est un nombre entier positif ;
le ou les polymères aminoplaste-éther étant choisis parmi les polymères de structure (III) ou (IIIbis) suivantes : 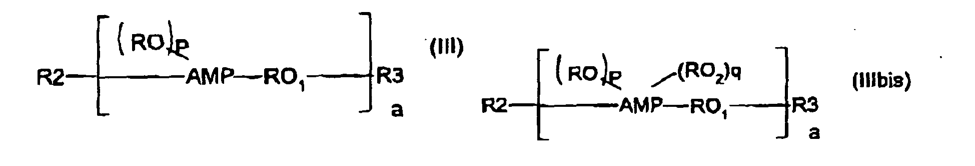 dans lesquelles :
AMP, R, RO₁, RO₂. p et q ont la même signification que ci-dessus,
R2 ou R3, identiques ou différents représentent un groupe terminal pouvant désigner un atome d'hydrogène, un groupement RO₁H, un groupement RO₂H, un groupement AMP(OR)p ou tout groupement monofonctionnel tel que alkyle, cycloalkyle, aryle, aralkyle, alkylaryle, alkyloxyalkyle, aryloxyalkyle, cycloalkoxyalkyle,
a étant un nombre plus grand que 1 et de préférence plus grand que 2;
les résidus aminoplastes porteurs de leurs groupements OR sont choisis parmi ceux de structure (XVI) suivante : 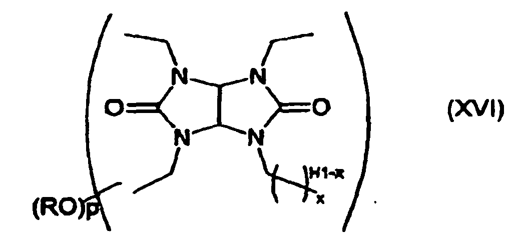 dans laquelle R, p, ont les mêmes significations que ci-dessus ; x désigne 0 ou 1 ;
les résidus alkylèneoxy sont ceux correspondants aux diols de formule générale (XVII) suivante:
HO)-(ZO)y(Z'O)y'-Z3OH (XVII),
y et y' étant des nombres allant de 0 à 1000,
et Z, Z' et Z3 désignent -CH2CH2-, l'un au moins de y ou y' étant différent de 0.

2. Composition selon la revendication 1.
**caractérisée par le fait que** les polymères à squelette aminoplaste-éther sont choisis parmi les produits suivants:
PEG-180/Octoxynol-40/TMMG Copolymer,
PEG-180/Laureth-50/TMMG Copolymer,
Polyether-1.

3. Composition selon la revendication 1 ou 2, **caractérisée par le fait que** les polymères à squelette aminoplaste-éther sont utilisés en une quantité pouvant varier de 0.01 à 10% en poids du poids total de la composition.

4. Composition selon la revendication 3, **caractérisée par le fait que** les polymères à squelette aminoplaste-éther sont utilisés en une quantité pouvant varier de 0,1 à 5% en poids du poids total de la composition.

5. Composition selon l'une quelconque des revendications précédentes, **caractérisée par le fait que** le ou les agents oxydants sont choisis parmi le peroxyde d'hydrogène et les composés libérant du peroxyde d'hydrogène par hydrolyse tels que le peroxyde d'urée et les persels, les chlorites, les systèmes enzymatiques générateurs d'espèces oxydantes et les peroxydes organiques.

6. Composition selon la revendication 5, **caractérisée par le fait que** le persel est un persulfate ou un perborate de métal alcalin ou alcalino-terreux.

7. Composition selon la revendication 5, **caractérisée par le fait que** le système enzymatique est une oxydoréductase à deux électrons associée à son donneur en présence d'air.

8. Composition selon la revendication 7, **caractérisée par le fait que** l'oxydoréductase est l'uricase et son donneur l'acide urique.

9. Composition selon la revendication 5, **caractérisée par le fait que** la concentration en peroxyde d'hydrogène varie de 2 à 40 volumes.

10. Composition selon la revendication 5, **caractérisée par le fait que** la concentration en composés oxydants peut varier de 0,1 à 25 % en poids par rapport au poids total de la composition.

11. Composition selon l'une quelconque des revendications précédentes, **caractérisée par le fait qu'**elle contient en outre des colorants directs.

12. Composition selon l'une quelconque des revendications précédentes, **caractérisée par le fait qu'**elle contient en outre au moins un polymère cationique ou amphotère.

13. Composition selon la revendication 12, **caractérisée par le fait que** le polymère cationique est un polymère de polyammonium quaternaire constitué de motifs récurrents répondant à la formule (W) suivante :

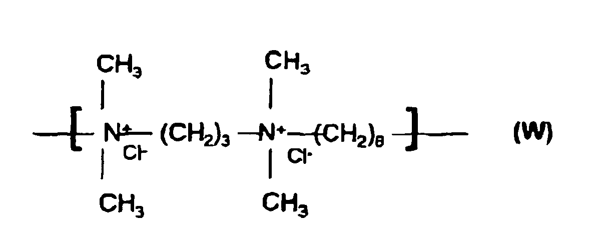

14. Composition selon la revendication 12, **caractérisée par le fait que** le polymère cationique est un polymère de polyammonium quaternaire constitué de motifs récurrents répondant à la formule (U) suivante :

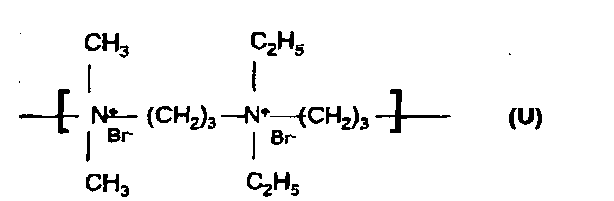

15. Composition selon la revendication 12, **caractérisée par le fait que** le polymère amphotère est un copolymère comprenant au moins comme monomères de l'acide acrylique et un sel de diméthyldiallylammonium.

16. Composition selon l'une quelconque des revendications 12 à 15, **caractérisée par le fait que** le ou les polymères cationiques ou amphotères représentent de 0,01 % à 10 %, de préférence de 0,05 % à 5 %, et encore plus préférentiellement de 0,1 % à 3 % en poids, du poids total de la composition.

17. Composition selon l'une quelconque des revendications précédentes, **caractérisée par le fait qu'**elle contient au moins un tensioactif choisi parmi les tensioactifs anioniques, cationiques, non ioniques ou amphotères.

18. Composition selon la revendication 17, **caractérisée par le fait que** les tensioactifs représentent 0,01 à 40% et de préférence de 0,1 à 30% en poids, du poids total de la composition.

19. Composition selon l'une quelconque des revendications précédentes, **caractérisée par le fait qu'**elle contient au moins un agent épaississant additionnel.

20. Composition selon la revendication 19, **caractérisée par le fait que** l'agent épaississant additionnel est un dérivé de cellulose, un dérivé de guar, une gomme d'origine microbienne, un épaississant synthétique.

21. Composition selon la revendication 19 ou 20, **caractérisée par le fait que** le
ou les agents épaississants additionnels représentent 0,05 à 10% en poids du poids total de la composition.

22. Composition selon l'une quelconque des revendications précédentes, **caractérisée par le fait qu'**elle contient en outre au moins un agent alcalin dans des quantités allant de 0,05 à 30% en poids du poids total de la composition.

23. Composition selon la revendication 22, **caractérisée par le fait que** l'agent alcalinisant est choisi parmi, l'ammoniaque, le chlorure d'ammonium, les carbonates alcalins ou alcalino-terreux, les silicates alcalins ou alcalino-terreux, les phosphates alcalins ou alcalino-terreux, les alcanolamines telles que les mono-, dl- et triéthanolamines ainsi que leurs dérivés, les hydroxydes de sodium ou de potassium et les composés de formule (XIX) suivante : 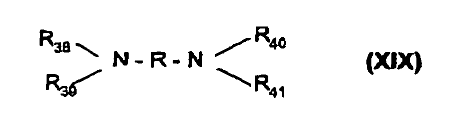 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

24. Composition selon l'une quelconque des revendications 1 à 23, **caractérisée par le fait qu'**elle est obtenue par mélange extemporané au moment de l'emploi d'une composition anhydre contenant au moins un agent oxydant et d'au moins une composition aqueuse, l'une au moins des compositions anhydre ou aqueuse contenant au moins un polymère à squelette aminoplaste-éther.

25. Composition selon la revendication 24, **caractérisée par le fait que** la composition anhydre est sous forme pulvérulente.

26. Composition selon la revendication 24 ou 25, **caractérisée par le fait que** la composition anhydre contient au moins un additif choisi parmi les charges minérales ou organiques telles que la silice ou les argiles, les liants tels que la vinylpyrrolidone, les huiles ou les cires, les polyalkylèneglycols ou les dérivés de polyalkylèneglycols, les lubrifiants comme les stéarates de polyol ou les stéarates de métaux alcalins ou alcalino-terreux et les agents colorants ou matifiants comme les oxydes de titane.

27. Composition selon la revendication 26, **caractérisée par le fait que** chacun des additifs peut être présent à une concentration allant de 0 à 30% en poids du poids total de la composition.

28. Composition selon l'une quelconque des revendications 1 à 23 ; **caractérisée par le fait qu'**elle est aqueuse.

29. Composition selon la revendication 28, **caractérisée par le fait que** le milieu aqueux contient un ou plusieurs solvants organiques.

30. Composition selon la revendication 29, **caractérisée par le fait que** la concentration en solvant(s) varie de 0,5 à 20% et, de préférence, de 2 à 10% en poids par rapport au poids total de la composition.

31. Composition selon l'une quelconque des revendications 28 à 30, **caractérisée par le fait que** le milieu aqueux contient du peroxyde d'hydrogène.

32. Composition selon l'une quelconque des revendications précédentes, **caractérisée par le fait que** la composition possède un pH allant de 4 à 12, de préférence de 7 à 11.5 et plus préférentiellement de 8 à 11.

33. Composition selon la revendication 24, **caractérisée par le fait que** le polymère à squelette aminoptaste-éther se trouve dans une composition aqueuse.

34. Procédé de décoloration des fibres kératiniques, en particulier des fibres kératiniques humaines telles que les cheveux, consistant à appliquer sur les fibres, sèches ou humides, une composition prête à remploi contenant, dans un milieu approprié pour la décoloration, au moins un agent oxydant et au moins un polymère à squelette aminoplaste-éther défini dans la revendication 1 ou 2, et à la laisser agir pendant un temps de pause variant de 1 à 60 minutes, et de préférence de 10 à 45 minutes, à rincer les fibres, éventuellement à les laver au shampooing, puis à les rincer à nouveau, et à les sécher.

35. Dispositif à deux compartiments ou « Kit » pour la décoloration des fibres kératiniques et en particulier des fibres kératiniques humaines telles que les cheveux, **caractérisé par le fait que** le premier compartiment contient au moins une poudre ou une composition aqueuse, et le deuxième compartiment une composition aqueuse, l'un au moins des deux compartiments contenant au moins un agent oxydant et l'un au moins des deux compartiments contenant au moins un polymère à squelette aminoplaste-éther.

## Claims

1. Ready-to-use composition for bleaching keratin fibres, in particular human keratin fibres such as the hair, comprising, in a medium that is suitable for bleaching, at least one oxidizing agent, **characterized in that** it also contains at least one thickening polymer with an aminoplast-ether skeleton chosen from those containing at least one unit of structure (I) below: 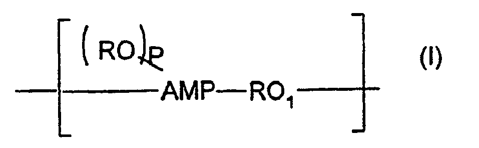 in which:
AMP is an aminoplast residue with alkylene units,
R denotes a hydrogen atom, a C₁-C₄ alkyl radical or a C₁-C₄ acyl radical,
RO₁ is a divalent alkyleneoxy residue,
p denotes a positive integer,
the group(s) OR being linked to the alkylene units of the AMP residue; and
those containing at least one unit of structure (II) below: 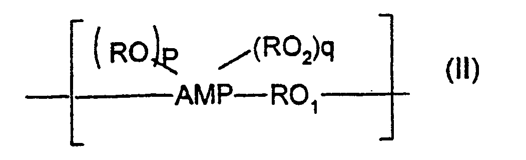
in which:
AMP, R, RO₁ and p have the same meaning as above,
RO₂ is a hydrophobic group other than RO linked to AMP via a hetero atom and comprising at least two carbon atoms, and
- q is a positive integer;
the aminoplast-ether polymer(s) being chosen from the polymers of structure (III) or (IIIa) below: 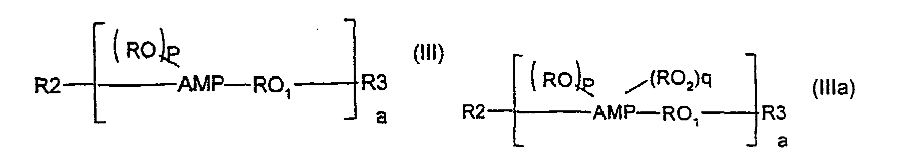
in which:
AMP, R, RO₁, RO₂, p and q have the same meaning as above,
R2 or R3, which may be identical or different, represent an end group that can denote a hydrogen atom,
a group RO₁H, a group RO₂H, a group AMP (OR) p or any monofunctional group such as alkyl, cycloalkyl, aryl, aralkyl, alkylaryl, alkyloxyalkyl, aryloxyalkyl or cycloalkoxyalkyl,
a being a number greater than 1 and preferably greater than 2;
the aminoplast residues bearing the groups OR thereof are chosen from those of structure (XVI) below: 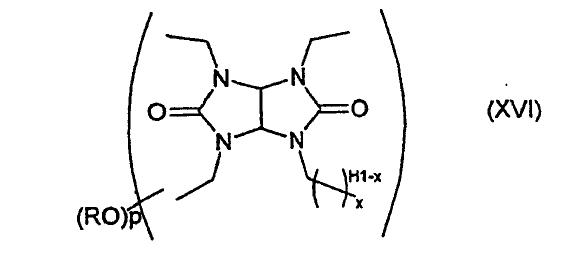
in which R and p have the same meanings as above;
x denotes 0 or 1;
the alkyleneoxy residues are those corresponding to the diols of general formula (XVII) below:
HO-(ZO)y-(Z'O)y'-Z3OH (XVII),
y and y' being numbers ranging from 0 to 1000, and Z, Z' and Z3 denote -CH2CH2-, at least one from among y and y' being other than 0.

2. Composition according to Claim 1,
**characterized in that** the polymers with an aminoplast-ether skeleton are chosen from the following products:
PEG-180/Octoxynol-40/TMMG Copolymer,
PEG-180/Laureth-50/TMMG Copolymer,
Polyether-1.

3. Composition according to Claim 1 or 2,
**characterized in that** the polymers with an aminoplast-ether skeleton are used in an amount that can range from 0.01% to 10% by weight relative to the total weight of the composition.

4. Composition according to Claim 3,
**characterized in that** the polymers with an aminoplast-ether skeleton are used in an amount that can range from 0.1% to 5% by weight relative to the total weight of the composition.

5. Composition according to any one of the preceding claims, **characterized in that** the oxidizing agent(s) is (are) chosen from hydrogen peroxide and compounds that release hydrogen peroxide by hydrolysis, such as urea peroxide and persalts, chlorites, enzymatic systems that generate oxidizing species and organic peroxides.

6. Composition according to Claim 5, **characterized in that** the persalt is an alkali metal or alkaline-earth metal persulfate or perborate.

7. Composition according to Claim 5, **characterized in that** the enzymatic system is a two-electron oxidoreductase combined with its donor in the presence of air.

8. Composition according to Claim 7, **characterized in that** the oxidoreductase is uricase and its donor is uric acid.

9. Composition according to Claim 5, **characterized in that** the hydrogen peroxide concentration ranges from 2 to 40 volumes.

10. Composition according to Claim 5, **characterized in that** the concentration of oxidizing compounds can range from 0.1% to 25% by weight relative to the total weight of the composition.

11. Composition according to any one of the preceding claims, **characterized in that** it also contains direct dyes.

12. Composition according to any one of the preceding claims, **characterized in that** it also contains at least one cationic or amphoteric polymer.

13. Composition according to Claim 12,
**characterized in that** the cationic polymer is a poly(quaternary ammonium) polymer consisting of repeating units corresponding to formula (W) below:

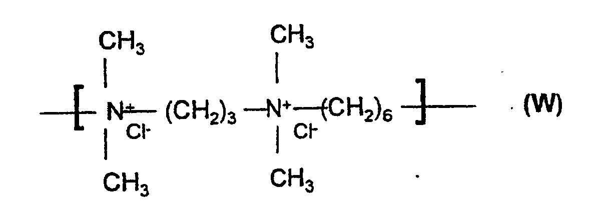

14. Composition according to Claim 12,
**characterized in that** the cationic polymer is a poly(quaternary ammonium) polymer consisting of repeating units corresponding to formula (U) below:

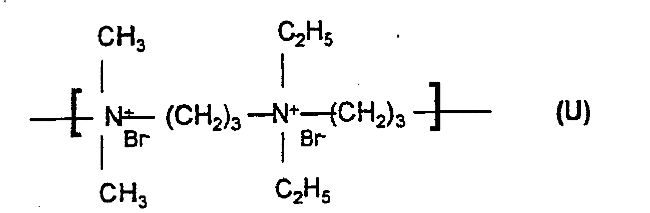

15. Composition according to Claim 12,
**characterized in that** the amphoteric polymer is a copolymer comprising at least, as monomers, acrylic acid and a dimethyldiallylammonium salt.

16. Composition according to any one of Claims 12 to 15, **characterized in that** the cationic or amphoteric polymer(s) represent(s) from 0.01% to 10%, preferably from 0.05% to 5% and even more preferably from 0.1% to 3% by weight relative to the total weight of the composition.

17. Composition according to any one of the preceding claims, **characterized in that** it contains at least one surfactant chosen from anionic, cationic, nonionic and amphoteric surfactants.

18. Composition according to Claim 17,
**characterized in that** the surfactants represent 0.01% to 40% and preferably 0.1% to 30% by weight relative to the total weight of the composition.

19. Composition according to any one of the preceding claims, **characterized in that** it contains at least one additional thickener.

20. Composition according to Claim 19,
**characterized in that** the additional thickener is a cellulose derivative, a guar derivative, a gum of microbial origin or a synthetic thickener.

21. Composition according to Claim 19 or 20,
**characterized in that** the additional thickener(s) represent(s) 0.05% to 10% by weight relative to the total weight of the composition.

22. Composition according to any one of the preceding claims, **characterized in that** it also contains at least one alkaline agent in amounts ranging from 0.05% to 30% by weight relative to the total weight of the composition.

23. Composition according to Claim 22, **characterized in that** the basifying agent is chosen from aqueous ammonia, ammonium chloride, alkali metal or alkaline-earth metal carbonates, alkali metal or alkaline-earth metal silicates, alkali metal or alkaline-earth metal phosphates, alkanolamines such as monoethanolamine, diethanolamine and triethanolamine and also derivatives thereof, sodium hydroxide, potassium hydroxide and the compounds of formula (XIX) below: 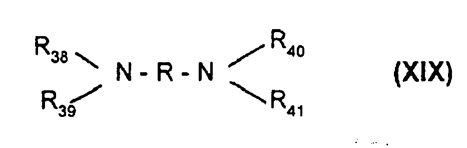 in which R is a propylene residue optionally substituted with a hydroxyl group or a C₁-C₄ alkyl radical; R₃₈, R₃₉, R₄₀ and R₄₁, which may be identical or different, represent a hydrogen atom, a C₁-C₄ alkyl radical or a C₁-C₄ hydroxyalkyl radical.

24. Composition according to any one of Claims 1 to 23, **characterized in that** it is obtained by extemporaneous mixing, at the time of use, of an anhydrous composition containing at least one oxidizing agent and of at least one aqueous composition, at least one of the anhydrous and aqueous compositions containing at least one polymer with an aminoplast-ether skeleton.

25. Composition according to Claim 24, **characterized in that** the anhydrous composition is in pulverulent form.

26. Composition according to Claim 24 or 25,
**characterized in that** the anhydrous composition contains at least one additive chosen from mineral or organic fillers such as silica or clays, binders such as vinylpyrrolidone, oils or waxes, polyalkylene glycols or polyalkylene glycol derivatives, lubricants, for instance polyol stearates or alkali metal or alkaline-earth metal stearates and colorants or matt-effect agents, for instance titanium oxides.

27. Composition according to Claim 26, **characterized in that** each of the additives may be present in a concentration ranging from 0% to 30% by weight relative to the total weight of the composition.

28. Composition according to any one of Claims 1 to 23, **characterized in that** it is aqueous.

29. Composition according to Claim 28, **characterized in that** the aqueous medium contains one or more organic solvents.

30. Composition according to Claim 29, **characterized in that** the solvent concentration ranges from 0.5% to 20% and preferably from 2% to 10% by weight relative to the total weight of the composition.

31. Composition according to any one of Claims 28 to 30, **characterized in that** the aqueous medium contains hydrogen peroxide.

32. Composition according to any one of the preceding claims, **characterized in that** the composition has a pH ranging from 4 to 12, preferably from 7 to 11.5 and more preferably from 8 to 11.

33. Composition according to Claim 24, **characterized in that** the polymer with an aminoplast-ether skeleton is in an aqueous composition.

34. Process for bleaching keratin fibres, in particular human keratin fibres such as hair, consisting in applying to wet or dry fibres a ready-to-use composition which contains, in a medium that is suitable for bleaching, at least one oxidizing agent and at least one polymer with an aminoplast-ether skeleton defined in Claim 1 or 2, and in leaving the composition to act for an exposure time ranging from 1 to 60 minutes, and preferably from 10 to 45 minutes, rinsing the fibres, and then optionally washing them with shampoo, followed by rinsing them again and drying them.

35. Two-compartment device or "kit" for bleaching keratin fibres and in particular human keratin fibres such as the hair, **characterized in that** the first compartment contains at least one powder or an aqueous composition, and the second compartment contains an aqueous composition, at least one of the two compartments containing at least one oxidizing agent and at least one of the two compartments containing at least one polymer with an aminoplast-ether skeleton.

## Patentansprüche

1. Gebrauchsfertige Zusammensetzung für die Entfärbung von Keratinfasern und insbesondere menschlichen Keratinfasern, wie Haaren, die in einem zum Entfärben geeigneten Medium mindestens ein Oxidationsmittel enthält, **dadurch gekennzeichnet, dass** sie ferner mindestens ein verdickendes Polymer mit Aminoplast-Ether-Grundstruktur enthält, das unter den Polymeren, die mindestens eine Einheit der folgenden Struktur (I) enthalten: 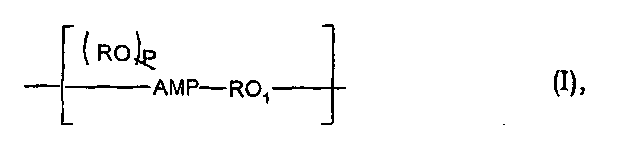 worin bedeuten:
- AMP einen Aminoplastrest mit Alkyleneinheiten,
- R ein Wasserstoffatom, eine C₁-₄-Alkylgruppe oder eine C₁₋₄-Acylgruppe,
- RO₁ einen zweiwertigen Alkylenoxyrest,
- p eine positive ganze Zahl,
wobei die Gruppe(n) OR an die Alkyleneinheiten des AMP-Rests gebunden sind; und
unter den Polymeren ausgewählt ist, die mindestens eine Einheit der folgenden Struktur (II) enthalten: 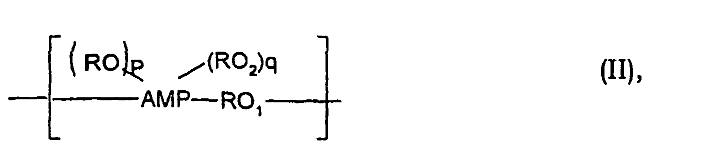 worin bedeuten:
- AMP, R, RO₁ und p die oben angegebenen Bedeutungen,
- RO₂ eine hydrophobe Gruppe, die von RO verschieden ist und über ein Heteroatom an AMP gebunden ist und mindestens zwei Kohlenstoffatome aufweist, und
- q eine positive ganze Zahl;
wobei das oder die Aminoplast-Ether-Polymer(e) unter den Polymeren der folgenden Strukturen (III) oder (IIIbis) ausgewählt sind: 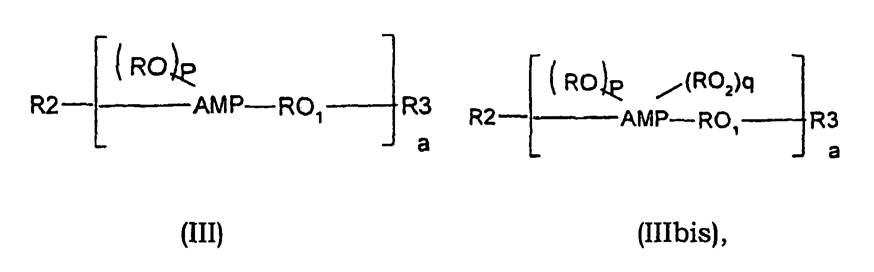 worin bedeuten:
AMP, R, RO₁, RO₂, p und q die oben angegebenen Bedeutungen,
R2 oder R3, die gleich oder verschieden sind, eine endständige Gruppe, die ein Wasserstoffatom, eine Gruppe RO₁H, eine Gruppe RO₂H, eine Gruppe AMP(OR)p oder beliebige monofunktionelle Gruppen bedeuten kann, wie Alkyl, Cycloalkyl, Aryl, Aralkyl, Alkylaryl, Alkyloxyalkyl, Aryloxyalkyl, Cycloalkoxyalkyl,
wobei a eine Zahl > 1 und vorzugsweise >2 bedeutet;
wobei die Aminoplastreste, die OR-Gruppen tragen, unter den
Resten der folgenden Struktur (XVI) ausgewählt sind: 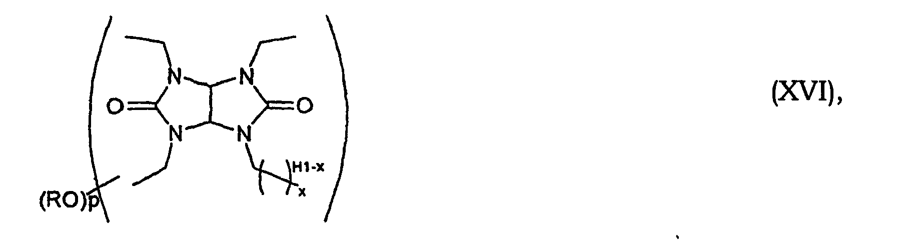 worin R und p oben angegebenen Bedeutungen aufweisen und x 0 oder 1 bedeutet;
wobei die Alkylenoxyreste Gruppen sind, die Diolen der allgemeinen Formel (XVII) entsprechen:
HO-(ZO)y-(Z'O)y'-Z3OH (XVII),
wobei y und y' Zahlen von 0 bis 1 000 bedeuten,
und Z, Z' und Z3 -CH2CH2- bedeuten, wobei mindestens ein
Wert y oder y' von 0 verschieden ist.

2. Zusammensetzung nach Anspruch 1, **dadurch gekennzeichnet, dass** die Polymere mit Aminoplast-Ether-Grundgerüst unter den folgenden Produkten ausgewählt sind:
PEG-180/Octoxynol-40/TMMG Copolymer,
PEG-180/Laureth-50/TMMG Copolymer,
Polyether-1.

3. Zusammensetzung nach Anspruch 1 oder 2, **dadurch gekennzeichnet, dass** die Polymere mit Aminoplast-Ether-Grundgerüst in einer Menge verwendet werden, die im Bereich von 0,01 bis 10 Gew.-% des Gesamtgewichts der Zusammensetzung liegen kann.

4. Zusammensetzung nach Anspruch 3, **dadurch gekennzeichnet, dass** die Polymere mit Aminoplast-Ether-Grundgerüst in einer Menge verwendet werden, die im Bereich von 0,1 bis 5 Gew.-% des Gesamtgewichts der Zusammensetzung liegen kann.

5. Zusammensetzung nach einem der vorhergehenden Ansprüche, **dadurch gekennzeichnet, dass** das oder die Oxidationsmittel unter Wasserstoffperoxid und Verbindungen, die durch Hydrolyse Wasserstoffperoxid bilden können, wie Harnstoffperoxid, Salzen von Persäuren, Chloriten, enzymatischen Systemen, die oxidierende Spezies bilden können, und organischen Peroxiden ausgewählt sind.

6. Zusammensetzung nach Anspruch 5, **dadurch gekennzeichnet,**
**dass** das Persalz ein Alkali- oder Erdalkalipersulfat oder ein Alkali- oder Erdalkaliperborat ist.

7. Zusammensetzung nach Anspruch 5, **dadurch gekennzeichnet, dass** das enzymatische System eine Oxidoreductase (2 Elektronen) in Kombination mit ihrem Donor in Gegenwart von Luft ist.

8. Zusammensetzung nach Anspruch 7, **dadurch gekennzeichnet, dass** die Oxidoreductase die Uricase und ihr Donor die Harnsäure ist.

9. Zusammensetzung nach Anspruch 5, **dadurch gekennzeichnet, dass** die Wasserstoffperoxid-Konzentration im Bereich von 2 bis 40 Volumina liegt.

10. Zusammensetzung nach Anspruch 5, **dadurch gekennzeichnet, dass** die Konzentration der oxidierenden Verbindungen im Bereich von 0,1 bis 25 Gew.-%, bezogen auf das Gesamtgewicht der Zusammensetzung, liegen kann.

11. Zusammensetzung nach einem der vorhergehenden Ansprüche, **dadurch gekennzeichnet, dass** sie ferner mindestens einen Direktfarbstoff enthält.

12. Zusammensetzung nach einem der vorhergehenden Ansprüche, **dadurch gekennzeichnet, dass** sie ferner mindestens ein kationisches oder amphoteres Polymer enthält.

13. Zusammensetzung nach Anspruch 12, **dadurch gekennzeichnet, dass** das kationische Polymer ein quartäres Polyammoniumpolymer ist, das aus wiederkehrenden Einheiten der folgenden Formel (W) besteht:

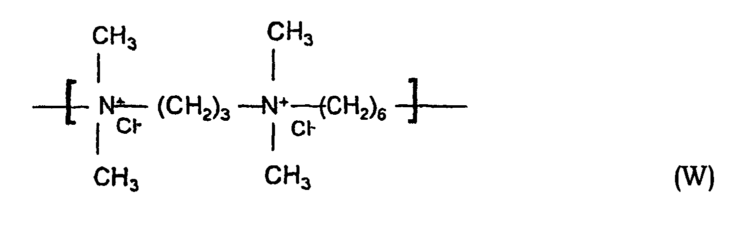

14. Zusammensetzung nach Anspruch 12, **dadurch gekennzeichnet, dass** das kationische Polymer ein quartäres Polyammoniumpolymer ist, das aus wiederkehrenden Einheiten der folgenden Formel (U) besteht:

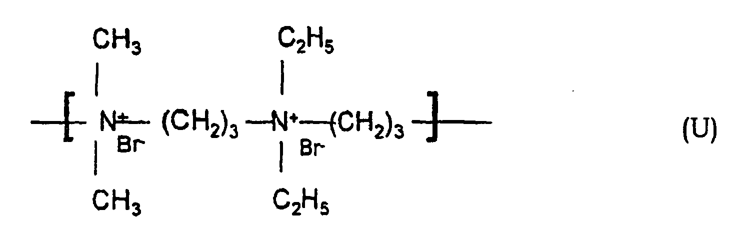

15. Zusammensetzung nach Anspruch 12, **dadurch gekennzeichnet, dass** das amphotere Polymer ein Copolymer ist, das als Monomere zumindest Acrylsäure und ein Dimethyldiallylammoniumsalz enthält.

16. Zusammensetzung nach einem der Ansprüche 12 bis 15, **dadurch gekennzeichnet, dass** das oder die kationische(n) oder amphotere(n) Polymer(e) 0,01 bis 10 %, vorzugsweise 0,05 bis 5 % und noch bevorzugter 0,1 bis 3 Gew.-% des Gesamtgewichts der Zusammensetzung ausmachen.

17. Zusammensetzung nach einem der vorhergehenden Ansprüche, **dadurch gekennzeichnet, dass** sie mindestens einen grenzflächenaktiven Stoff enthält, der unter den anionischen, kationischen, nichtionischen oder amphoteren grenzflächenaktiven Stoffen ausgewählt ist.

18. Zusammensetzung nach Anspruch 17, **dadurch gekennzeichnet, dass** die grenzflächenaktiven Stoffe 0,01 bis 40 % und vorzugsweise 0,1 bis 30 Gew.-% des Gesamtgewichts der Zusammensetzung ausmachen.

19. Zusammensetzung nach einem der vorhergehenden Ansprüche, **dadurch gekennzeichnet, dass** sie mindestens ein zusätzliches Verdickungsmittel enthält.

20. Zusammensetzung nach Anspruch 19, **dadurch gekennzeichnet, dass** das zusätzliche Verdickungsmittel ein Cellulosederivat, Guarderivat, Gummi mikrobieller Herkunft oder synthetisches Verdickungsmittel ist.

21. Zusammensetzung nach Anspruch 19 oder 20, **dadurch gekennzeichnet, dass** das oder die ergänzende(n) Verdickungsmittel 0,05 bis 10 Gew.-% des Gesamtgewichts der Zusammensetzung ausmachen.

22. Zusammensetzung nach einem der vorhergehenden Ansprüche, **dadurch gekennzeichnet, dass** sie ferner mindestens ein Alkalisierungsmittel in Mengenanteilen von 0,05 bis 30 % des Gesamtgewichts der Zusammensetzung enthält.

23. Zusammensetzung nach Anspruch 22, **dadurch gekennzeichnet, dass** das Alkalisierungsmittel unter Ammoniak, Ammoniumchlorid, Alkalicarbonaten, Erdalkalicarbonaten, Alkalisilicaten, Erdalkalisilicaten, Alkaliphosphaten, Erdalkaliphosphaten, Alkanolaminen, wie Mono-, Di- und Triethanolamin, sowie deren Derivaten, Natriumhydroxid oder Kaliumhydroxid und den Verbindungen der folgenden Formel (XIX) ausgewählt ist, 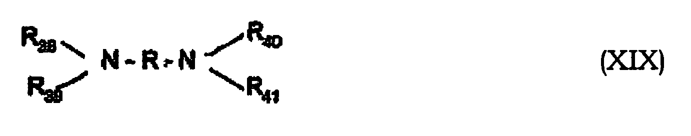 worin R eine gegebenenfalls mit einer Hydroxygruppe oder einer C₁₋₄-Alkylgruppe substituierte Propylengruppe ist; und die Gruppen R₃₈, R₃₉, R₄₀ und R₄₁, die gleich oder verschieden sind, Wasserstoff, C₁₋₄-Alkyl oder C₁₋₄-Hydroxyalkyl bedeuten.

24. Zusammensetzung nach einem der Ansprüche 1 bis 23, **dadurch gekennzeichnet, dass** sie durch bedarfsgemäßes Mischen einer wasserfreien Zusammensetzung, die mindestens ein Oxidationsmittel enthält, und mindestens einer wässrigen Zusammensetzung bei der Anwendung hergestellt wird, wobei mindestens eine der Zusammensetzungen (wasserfrei oder wässrig) mindestens ein Polymer mit Aminoplast-Ether-Grundgerüst enthält.

25. Zusammensetzung nach Anspruch 24, **dadurch gekennzeichnet, dass** die wasserfreie Zusammensetzung pulverförmig vorliegt.

26. Zusammensetzung nach Anspruch 24 oder 25, **dadurch gekennzeichnet, dass** die wasserfreie Zusammensetzung mindestens einen Zusatzstoff enthält, der unter den anorganischen oder organischen Füllstoffen, wie Kieselsäure oder Tonen, Bindemitteln, wie Vinylpyrrolidon, Ölen und Wachsen, Polyalkylenglycolen oder Polyalkylenglycolderivaten, Gleitmitteln wie Polyolstearaten oder Alkali- oder Erdalkalistearaten, Farbmitteln oder Trübungsmitteln wie Titanoxiden ausgewählt ist.

27. Zusammensetzung nach Anspruch 26, **dadurch gekennzeichnet, dass** jeder Zusatzstoff in einer Konzentration von 0 bis 30 Gew.-% des Gesamtgewichts der Zusammensetzung enthalten sein kann.

28. Zusammensetzung nach einem der Ansprüche 1 bis 23, **dadurch gekennzeichnet, dass** sie wässrig ist.

29. Zusammensetzung nach Anspruch 28, **dadurch gekennzeichnet, dass** das wässrige Medium ein oder mehrere organische Lösungsmittel enthält.

30. Zusammensetzung nach Anspruch 29, **dadurch gekennzeichnet, dass** die Konzentration des Lösungsmittels oder der Lösungsmittel im Bereich von 0,5 bis 20 % und vorzugsweise 2 bis 10 Gew.-%, bezogen auf das Gesamtgewicht der Zusammensetzung, liegt.

31. Zusammensetzung nach einem der Ansprüche 28 bis 30, **dadurch gekennzeichnet, dass** das wässrige Medium Wasserstoffperoxid enthält.

32. Zusammensetzung nach einem der vorhergehenden Ansprüche, **dadurch gekennzeichnet, dass** die Zusammensetzung einen pH-Wert von 4 bis 12, vorzugsweise 7 bis 11,5 und noch bevorzugter 8 bis 11 aufweist.

33. Zusammensetzung nach Anspruch 24, **dadurch gekennzeichnet, dass** das Polymer mit Aminoplast-Ether-Grundgerüst in der wässrigen Zusammensetzung enthalten ist.

34. Verfahren zum Entfärben von Keratinfasern und insbesondere menschlichen Keratinfasern wie Haaren, das darin besteht, auf die trockenen oder feuchten Fasern eine gebrauchsfertige Zusammensetzung aufzutragen, die in einem zum Entfärben geeigneten Medium mindestens ein Oxidationsmittel und mindestens ein in Anspruch 1 oder 2 definiertes Polymer mit Aminoplast-Ether-Grundgerüst enthält, und während einer Zeitspanne von 1 bis 60 min und vorzugsweise 10 bis 45 min einwirken zu lassen, die Fasern zu spülen, gegebenenfalls mit Haarwaschmittel zu waschen, nochmals zu spülen und die Fasern zu trocknen.

35. Vorrichtung mit 2 Abteilungen oder Kit zum Entfärben von Keratinfasern und insbesondere menschlichen Keratinfasern wie Haaren, **dadurch gekennzeichnet, dass** die erste Abteilung mindestens ein Pulver oder eine wässrige Zusammensetzung enthält und die zweite Abteilung eine wässrige Zusammensetzung enthält, wobei mindestens eine Abteilung mindestens ein Oxidationsmittel und mindestens eine Abteilung mindestens ein Polymer mit Aminoplast-Ether-Grundgerüst enthält.
